(19) Europäisches Patentamt
European Patent Office
Office européen des brevets

(11) Veröffentlichungsnummer: **0 272 472 B1**

(12) # EUROPÄISCHE PATENTSCHRIFT

(45) Veröffentlichungstag der Patentschrift: **21.08.91**

(51) Int. Cl.⁵: **A61K 7/09**, C08L 33/10, C08L 5/08

(21) Anmeldenummer: **87117270.6**

(22) Anmeldetag: **24.11.87**

(54) **Kosmetisches Mittel auf der Basis von Chitosan und ampholytischen Copolymerisaten sowie ein neues Chitosan/Polyampholyt-Salz.**

(30) Priorität: **23.12.86 DE 3644097**

(43) Veröffentlichungstag der Anmeldung:
**29.06.88 Patentblatt 88/26**

(45) Bekanntmachung des Hinweises auf die Patenterteilung:
**21.08.91 Patentblatt 91/34**

(84) Benannte Vertragsstaaten:
**AT BE CH DE ES FR GB GR IT LI NL SE**

(56) Entgegenhaltungen:
**EP-A- 0 002 506**
**EP-A- 0 020 183**
**GB-A- 2 026 517**

(73) Patentinhaber: **Wella Aktiengesellschaft**
**Berliner Allee 65**
**W-6100 Darmstadt(DE)**

(72) Erfinder: **Lang, Günther, Dr.**
**Auf der Roten Erde 10B**
**W-6107 Reinheim 5(DE)**
Erfinder: **Wendel, Harald**
**Grabengasse 3**
**W-6105 Ober-Ramstadt(DE)**

**EP 0 272 472 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft ein kosmetisches Mittel auf der Basis von wäßrigen Chitosan/Polyampholyt-Mischungen beziehungsweise wasserlöslichen Chitosan/Polyampholyt-Salzen, welche durch Kombination von Chitosan mit bestimmten ampholytischen Copolymerisaten erhältlich sind.

Chitosan ist ein durch Deacetylierung von Chitin herstellbares Polyglucosamin, welches bereits für die Verwendung in zahlreichen kosmetischen Mitteln vorgeschlagen worden ist. Hierbei wird das Chitosan insbesondere in Form seiner wasserlöslichen Salze mit organischen Säuren oder Aniontensiden eingesetzt. In diesem Zusammenhang wird Bezug genommen auf die eigene europäische Patentschrift 0 002 506, die eigene deutsche Patentschrift 26 27 419 sowie die eigene deutsche Offenlegungsschrift 31 40 134.

Die bisher verwendeten Chitosansalze neigen jedoch aufgrund ihres stark ionischen, salzartigen Charakters dazu, trübe, spröde und wenig haftende Filme zu bilden. Aus diesem Grunde zeigen Festiger mit einem, für eine starke Festigung notwendigen, hohen Gehalt an diesen Chitosansalzen eine kosmetisch unbefriedigende, starke Abschuppung des Chitosansalz-Films vom Haar, so daß eine Festigung von schwer zu festigendem Haar auf diesem Wege nicht möglich ist. Außerdem ist bekannt, daß Salze des Chitosans mit mehrwertigen Anionen, wie zum Beispiel Sulfat oder Phosphat, in Wasser unlöslich oder nur wenig löslich sind. Auch oder Alginsäure, bildet Chitosan in Wasser unlösliche Polysalze.

Aufgabe der Erfindung ist es daher, ein kosmetisches Mittel auf der Basis von Chitosan zur Verfügung zu stellen, mit dem sich die vorstehend beschriebenen Naohteile bezüglich der Filmeigenschaften beziehungsweise der Löslichkeit vermeiden lassen.

Überraschend wurde nun gefunden, daß Salze des Chitosans mit bestimmten, aus der offengelegten britischen Patentanmeldung 2 104 091 sowie der deutschen Offenlegungsschrift 28 29 018 bekannten, ampholytischen Copolymerisaten wasserlöslich sind, beziehungsweise das Chitosan in wäßrigen Lösungen dieser Polyampholyte gelöst werden kann. Durch die Kombination der beiden Verbindungen werden darüberhinaus die vorteilhaften filmbildenden Eigenschaften eines bekannten synthetischen Polymers mit denen bekannter Chitosansalze verbunden, ohne die Nachteile der beiden Ausgangsstoffe zu übernehmen (siehe hierzu auch Beispiel 2).

Gegenstand der vorliegenden Erfindung ist daher ein kosmetisches Mittel zur Behandlung der Haare und/oder der Haut auf der Basis von 0,05 bis 10 Gewichtsprozent Chitosan, welches durch einen Gehalt an 0,05 bis 20 Gewichtsprozent eines ampholytischen Copolymerisates der Formel (I)

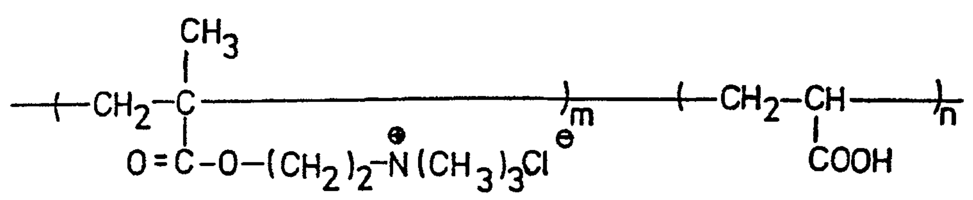
(I) ,

wobei m = 0,5 bis 0,9,
n = 0,1 bis 0,5 und
m + n = 1 ist,

gekennzeichnet ist.

Das ampholytische Copolymerisat der Formel (I) (im folgenden auch Polyampholyt genannt) sowie Verfahren zu dessen Herstellung werden in der offengelegten britischen Patentanmeldung 2 104 091 sowie in der deutschen Offenlegungsschrift 28 29 018 beschrieben. Die für eine Verwendung in den erfindungsgemäßen kosmetischen Mitteln geeigneten Polyampholyte der Formel (I) besitzen einen kationischen Anteil von 50 bis 90 Molprozent und eine Grenzviskositätszahl (ermittelt mit einem DIN-Ubbelohde-Kapillarviskosimeter bei 25 Grad Celsius in einer wäßrigen Lösung von 0,2 mol/l Essigsäure und 0,1 mol/l Natriumchlorid) von 3,3 bis 7,4 [ml/g].

Das Verhältnis des Gehalts an kationischen Monomereinheiten (a) und anionischen Monomereinheiten (b) bestimmt die Eigenschaften des ampholytischen Copolymerisates.

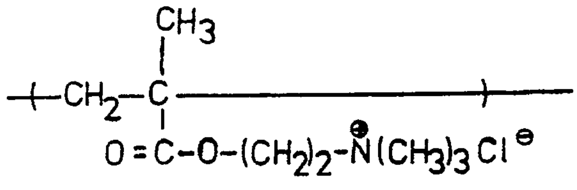

(a)

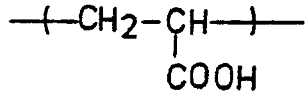

(b)

Insbesondere für die Löslichkeit des Chitosans in wäßrigen Lösungen des Polyampholyten beziehungsweise die Wasserlöslichkeit des gebildeten Chitosan/Polyampholyt-Salzes ist dies von besonderer Bedeutung. Die Löslichkeit des Chitosans in wäßrigen Lösungen des Polyampholytes steigt nämlich mit zunehmendem kationischen Anteil des Polyampholytes an (vergleiche Beispiel 1).

Besonders vorteilhaft sind solche Chitosan/Polyampholyt-Mischungen, bei denen ein Polyampholyt mit einer Grenzviskositätszahl von 4,6 bis 5,9 [ml/g](ermittelt in einer wäßrigen Lösung von 0,2 mol/l Essigsäure und 0,1 mol/l Natriumchlorid) sowie einem Gehalt an kationischen Monomereinheiten (a) von 70 bis 80 Molprozent und einem Gehalt an anionischen Monomereinheiten (b) von 20 bis 30 Molprozent verwendet wird.

Das molare Verhältnis von Chitosan (bezogen auf seinen Gehalt an freien Aminogruppen) und ampholytischem Copolymerisat (bezogen auf seinen anionischen Anteil) beträgt in dem erfindungsgemäßen kosmetischen Mittel vorzugsweise 1 : 1 bis 1 : 5.

Das erfindungsgemäße, eine Chitosan/Polyampholyt-Mi-schung beziehungsweise ein Chitosan/Polyampholyt-Salz enthaltende Mittel eignet sich ganz allgemein zur Behandlung der Haare und/oder der Haut. Es kann insbesondere vorliegen als Haar- und/oder Körperwaschmittel, Tönungsshampoo, Frisiercreme, Frisierlotion, Fönlotion, Mittel zur Festigung der Frisur, Waschlotion, Haarkur, Mittel zur Färbung von Haaren, Mittel zum Auftragen vor oder nach der Haarfärbung und als kosmetisches Mittel zur Pflege, zum Schutz oder zur Reinigung der Haut. Beispiele für ein kosmetisches Mittel zur Pflege, zum Schutz oder zur Reinigung der Haut sind Gesichtswässer, Schaum- und Duschbad-Präparate, Feuchthaltecremes, Coldcremes, Körperlotionen, Sonnenschutzmittel oder auch Make-up-Präparate wie Abschminkmilchprodukte.

Das Chitosan und das ampholytische Copolymerisat der Formel (I) sind in dem erfindungsgemäßen kosmetischen Mittel in einer Gesamtmenge von etwa 0,1 bis 30 Gewichtsprozent, vorzugsweise 0,1 bis 10 Gewichtsprozent, enthalten.

Das kosmetische Mittel gemäß der vorliegenden Erfindung kann zusätzlich zu dem Gehalt an Chitosan und ampholytischem Copolymerisat zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Das erfindungsgemäße kosmetische Mittel weist in bevorzugter Weise einen pH-Wert von 2 bis 11 auf und kann in Form einer wäßrigen oder wäßrig-alkoholischen Zubereitung, insbesondere als Lösung, als Creme, als Gel, als Dispersion oder als Emulsion vorliegen.

Ebenfalls ist es möglich, dieses Mittel mit Hilfe eines Zerstäubers beziehungsweise anderer geeigneter Sprühvorrichtungen oder im Gemisch mit üblichen unter Druck verflüssigten Treibmitteln als Aerosolspray (zum Beispiel Aerosolhaarspray) oder Aerosolschaum aus einem Druckbehälter zu entnehmen.

In bevorzugter Weise handelt es sich bei dem erfindungsgemäßen kosmetischen Mittel um ein Mittel zur Festigung der Frisur, wie beispielsweise einen flüssigen Haarfestiger oder Haarspray. Dieses Mittel liegt üblicherweise als wäßrige oder wäßrig-alkoholische Lösung vor, die durch einen Gehalt an einer Chitosan/Polyampholyt-Mischung beziehungsweise einem Chitosan/Polyampholyt-Salz gekennzeichnet ist. Hierbei kann die Chitosan/Polyampholyt-Mischung beziehungsweise das Chitosan/Polyampholyt-Salz selbst als filmbildendes oder festigendes Harz eingesetzt werden; es können jedoch auch zusätzlich andere filmbildende natürliche oder synthetische kosmetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden zum Beispiel Polyvinylpyrrolidon, Polyvinylacetat, Polyacrylverbindungen, wie beispielsweise Acrylsäure- oder Methacrylsäurepolymerisate, basische Polymerisate von Estern aus Acrylsäure oder Methacrylsäure mit Aminoalkoholen oder die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Das Mittel weist dann insbesondere einen pH-Wert zwischen 6 und 8 auf. Ein solches Mittel zur Festigung der Frisur enthält üblicherweise die filmbildenden Polymere in einer Gesamtmenge von etwa 0,1 bis 6,0 Gewichtsprozent. Enthält das Mittel neben der hier beschriebenen Chitosan/Polyampholyt-Mischung beziehungsweise dem Chitosan/Polyampholyt-Salz noch andere filmbildende Polymere, so reduziert sich der Gehalt an der Chitosan/Polyampholyt-Mischung beziehungsweise dem Chitosan/Polyampholyt-Salz entsprechend.

Als Alkohole kommen insbesondere die für kosmetische Zwecke üblicherweise verwendeten niederen Alkohole mit 1 bis 4 Kohlenstoffatomen, wie zum Beispiel Ethanol und Isopropanol, in Betracht.

Wenn das Mittel zur Festigung der Frisur in Form eines Aerosolpräparates vorliegt, welches aus einem Druckbehälter versprüht wird, so enthält es in der Kosmetikgrundlage etwa 10 bis 60 Gewichtsprozent eines Treibmittels. Als Treibmittel können Chlorfluoralkane, wie zum Beispiel $CCl_3F$, $CCl_2F_2$, $C_2Cl_3F_3$, $(CCl_2F)_2$, $CHCl_2F$ und $(CClF_2)_2$, leichtflüchtige Kohlenwasserstoffe, wie zum Beispiel n-Butan und n-Propan, oder auch Dimethylether, Kohlendioxid, Distickstoffmonoxid, Stickstoff, Methylenchlorid und 1,1,1-Trichlorethan, Verwendung finden.

Das erfindungsgemäße Mittel zur Festigung der Frisur kann weiterhin die für solche Mittel üblichen Zusätze, wie beispielsweise Parfümöle, Bakterizide, Fungizide, kämmbarkeitsverbessernde Substanzen, Modifiziermittel, wie zum Beispiel Siliconöl, und Weichmacher, wie beispielsweise Isopropylmyristat, Phtalsäurediethylester und Diethylstearat, enthalten.

Das erfindungsgemäße Mittel zur Festigung der Frisur kann gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche für Haarfestiger bekannte, direkt auf das Haar aufziehende kosmetische Farbstoffe, wie beispielsweise aromatische Nitrofarbstoffe (zum Beispiel 1,4-Diamino-2-nitro-benzol, Pikraminsäure, 1-Hydroxy-2-amino-4-nitro-benzol und 1,4-Bis-[(2-hydroxyethyl)-amino]-2-nitro-5-chlor-benzol), Azofarbstoffe (zum Beispiel C.I. 14 805-Acid Brown 4), Anthrachinonfarbstoffe (zum Beispiel C.I. 61 105-Disperse Violet 4) und Triphenylmethanfarbstoffe (zum Beispiel C.I. 42 535-Basic Violet 1), wobei die Farbstoffe dieser Klassen je nach der Art ihrer Substituenten sauren, nichtionogenen oder basischen Charakter haben können. Die Gesamtkonzentration dieser Farbstoffe beträgt hierbei üblicherweise etwa 0,01 bis 2,0 Gewichtsprozent.

Das erfindungsgemäße Mittel zur Festigung der Frisur weist bei gleich guter Festigung des Haares gegenüber bekannten und üblichen Mitteln auf der Basis von Chitosan oder Chitosansalzen eine besonders gute Kämmbarkeit und einen guten Griff des Haares im nassen Zustand sowie einen besonders angenehmen Griff des Haares im getrockneten Zustand auf.

Weiterhin kann das erfindungsgemäße Mittel ein Haarwaschmittel darstellen. Es liegt dann in Form einer wäßrigen Lösung oder Emulsion vor und enthält neben etwa 0,5 bis 6 Gewichtsprozent Chitosan/Polyampholyt-Mischung beziehungsweise Chitosan/Polyampholyt-Salz zumindest ein anionisches, kationisches, nichtionisches oder amphoteres Tensid.

In diesem Haarwaschmittel beträgt die Konzentration des Tensides im allgemeinen etwa 3 bis 50 Gewichtsprozent, vorzugsweise 3 bis 25 Gewichtsprozent, wobei der pH-Wert im allgemeinen zwischen 3 und 9, vorzugsweise zwischen 4 und 7, liegt.

Das erfindungsgemäße Mittel, das in Form eines Haarwaschmittels vorliegt, enthält im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Kokosfettsäuren, Lauryl- oder Ölsäuremono- oder -diethanolamid, die zweckmäßigerweise in Mengen von 1 bis 10 und vorzugsweise von 1 bis 3 Gewichtsprozent Verwendung finden, angeführt werden.

Unter den Verdickern können insbesondere Acrylpolymere und Cellulosederivate, wie zum Beispiel Carboxymethylcellulose, Hydroxypropylmethylcellulose und Hydroxyethylcellulose, angeführt werden. Die Verdicker liegen im allgemeinen in einem Anteil von 0,1 bis 5 Gewichtsprozent vor.

Unter den Tensiden oder oberflächenaktiven Agenzien, die in Kombination mit den neuen Chitosan/Polyampholyt-Mischungen beziehungsweise Chitosan/Polyampholyt-Salzen verwendet werden, können beispielsweise die folgenden genannt werden:

a) die anionischen oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$ bis $C_{18}$-Alkyl- und insbesondere $C_{12}$ bis $C_{14}$-Alkyl-Sulfatnatriumsalzeoder -Triethanolaminsalze, die Natrium-oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl-und Stearylalkohol, allein oder im Gemisch; die Fettalkohole von oxethyliertem Lanolin oder oxethyliertes Lanolin; Polyglycolether von gesättigten oder ungesättigten Fettalkoholen und Alkylphenolen mit 8 bis 30 Kohlenstoffatomen im Alkylrest und 1 bis 10 Glycerileinheiten im Molekül, Fettsäurealkanolamide, Sorbitanfettsäureester sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, die Alkylamidethyltrimethylammoniumethersulfate, Verbindungen mit kationischem Charakter wie Aminoxide, beispielsweise Alkyldimethylaminoxide und Alkylaminoethyldimethylaminoxide, oxyethylalkylammoniumphosphate , Pentaoxyethylammoniumchloride oder Alkyldimethylammoniumsaccharinate;

d) die amphoteren oder zwitterionischen oberflächenaktiven Agenzien, wie beispielsweise die Carboxylderivate von Imidazol, die N-Alkylbetaine, die N-Alkylamidobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamido-stearat-betain.

Das erfindungsgemäße kosmetische Mittel kann auch eine Creme oder Lotion zur Verwendung als Haarkur oder Hautpflegemittel darstellen. Es liegt dann meist in Form einer Öl-in-Wasser- oder Wasser-in-Öl-Emulsion oder -Suspension vor und enthält zusätzlich zu der Chitosan/Polyampholyt-Mischung beziehungsweise dem Chitosan/Polyampholyt-Salz kationische, nichtionogene, amphotere oder anionische Emulgatoren sowie als Bestandteil der Ölphase zum Beispiel Fettalkohole, Fettsäureester oder -amide, weiterhin Parfümöle, Vaseline, Wollfettalkohol oder feste beziehungsweise flüssige Paraffine.

Wenn das erfindungsgemäße Mittel ein Haartönungs-oder Haarfärbemittel darstellt, so liegt es ebenfalls bevorzugt in Form einer Creme, einer Lotion oder eines Shampoos vor und enthält zusätzlich etwa 0,05 bis 2,0 Gewichtsprozent eines üblichen, direkt auf das Haar aufziehenden Haarfarbstoffes aus der Gruppe der aromatischen Nitrofarbstoffe, Azofarbstoffe, Anthrachinonfarbstoffe und Triphenylmethanfarbstoffe, wobei die Farbstoffe dieser Klassen je nach der Art ihrer Substituenten sauren, nichtionogenen oder basischen Charakter haben können. Weiterhin kann dieses Mittel gegebenenfalls Alkalisierungsmittel, Antioxidantien sowie weitere für solche Mittel übliche kosmetische Zusätze und Hilfsstoffe enthalten.

Wie bereits erwähnt wurde, kann das erfindungsgemäße kosmetische Mittel auch zur Behandlung der Haut verwendet werden.

Das erfindungsgemäße kosmetische Mittel liegt hierzu vorzugsweise in Form einer Creme, eines Gels, einer Emulsion oder einer wäßrigen oder wäßrig-alkoholischen Lösung vor, welche die Chitosan/Polyampholyt-Mischung beziehungsweise das Chitosan/Polyampholyt-Salz in einer Konzentration von etwa 0,1 bis 10 Gewichtsprozent und vorzugsweise von 0,2 bis 6 Gewichtsprozent enthält.

Die im allgemeinen in einer derartigen Kosmetikzubereitung enthaltenen Hilfsstoffe sind beispielsweise Duftstoffe, Farbstoffe, Konservierungsmittel, Verdickungsmittel, Sequestriermittel, Emulgiermittel, Sonnenschutzfilter und ähnliche.

Diese Zubereitung für die Hautbehandlung liegt insbesondere in Form einer Creme oder Lotion zur Pflege der Hände oder des Gesichts oder in Form einer Sonnenschutzcreme, einer gefärbten Creme, eines Abschminkmilchproduktes, eines Schaumbad- und Duschbad-Präparates oder auch in Form einer Desodorierzubereitung vor.

Eine derartige Zubereitung wird unter Anwendung klassischer Verfahrensweisen hergestellt. Beispielsweise kann man zur Bildung einer Creme eine wäßrige Phase, die die erfindungsgemäße Chitosan/Polyampholyt-Mischung beziehungsweise das Chitosan/Polyampholyt-Salz und gegebenenfalls andere Bestandteile oder Hilfsstoffe gelöst enthält, und eine ölige Phase emulgieren. Für die ölige Phase kann man verschiedenartige Verbindungen verwenden, beispielsweise Paraffinöl, Vaselinöl, Süßmandelöl, Avocadoöl, Olivenöl, Fettsäureester, wie zum Beispiel Glycerylmonstearat, Ethylpalmitat und Isopropylpalmitat, oder Alkylmyristate, wie zum Beispiel Propylmyristat, Butylmyristat und Cetylmyristat. Man kann sie auch mit Fettsäurealkoholen, beispielsweise Stearyl- oder Cetylalkohol, oder Wachsen, beispielsweise Bienenwachs oder Wollwachs versetzen.

Die Chitosan/Polyampholyt-Mischung beziehungsweise das Chitosan/Polyampholyt-Salz kann in dieser Kosmetikzubereitung für die Hautpflege sowohl als Hauptwirkstoff als auch als Hilfsstoff enthalten sein.

Das erfindungsgemäße Hautbehandlungsmittel beeinträchtigt nicht die natürliche Hautatmung und

erleichtert die Stabilisierung des Feuchtegehaltes der Haut. Weiterhin verleiht dieses Mittel der Haut einen weichen Griff und eine hervorragende Geschmeidigkeit.

Die in den erfindungsgemäßen kosmetischen Mitteln enthaltenen Chitosan/Polyampholyt-Mischungen beziehungsweise Chitosan/Polyampholyt-Salze sind durch Lösen von Chitosan in einer wäßrigen Lösung eines ampholytischen Copolymerisates der Formel (I) oder durch Versetzen einer wäßrigen Lösung eines wasserlöslichen Chitosansalzes mit einem ampholytischen Copolymerisat der Formel (I) erhältlich. Die Umsetzung kann hierbei sowohl bei etwa 20 bis 25 Grad Celsius als auch bei etwa 40 bis 60 Grad Celsius durchgeführt werden. Die Reaktionsdauer beträgt bei 20 bis 25 Grad Celsius etwa 8 bis 16 Stunden und bei 40 bis 60 Grad Celsius etwa 1 bis 2 Stunden. Gegebenenfalls werden nach Beendigung der Reaktion wasserunlösliche Bestandteile durch Filtration aus der Lösung entfernt.

Die zum Lösen des Chitosans, das heißt zur Bildung des Chitosan/Polyampholyt-Salzes, erforderliche Menge des Polyampholyten ist hierbei von der Höhe des anionischen Anteils (n) des Polyampholyten abhängig und kann aus dem titrimetisch ermittelten Säuregruppengehalt des Polyampholyten errechnet werden. Das molare Verhältnis von Chitosan (bezogen auf seinen Gehalt an freien Aminogruppen) und Polyampholyten (bezogen auf seinen anionischen Anteil) beträgt zweckmäßigerweise 1 : 1 bis 1 : 5, wobei ein äquimolares Verhältnis bevorzugt ist. Es kann jedoch auch ein geringfügiger Unterschuß oder ein beliebiger Überschuß des Polyampholyten eingesetzt werden.

Ein weiterer Gegenstand der vorliegenden Erfindung sind daher Chitosan/Polyampholyt-Salze, welche durch das vorstehend beschriebene Verfahren erhältlich sind.

Die folgenden Beispiele sollen den Gegenstand der Erfindung näher erläutern.

**Beispiele**

**Beispiel 1** Herstellung und Löslichkeit von Chitosan/Polyampholyt-Mischungen

Eine wäßrige Mischung, welche 1 Gewichtsprozent Chitosan und die zur Bildung eines 1 : 1 Salzes von Chitosan und Polyampholyt gemäß Tabelle 1 theoretisch erforderliche Menge eines Polyampholytes der Formel (I) enthält, wird über Nacht gerührt. Anschließend wird die Wasserlöslichkeit des gebildeten Chitosan/Polyampholyt-Salzes beziehungsweise die Löslichkeit des Chitosans in der Polyampholytlösung bestimmt.

**Beispiel 2** Vergleichsversuche

Die wäßrigen, Chitosan/Polyampholyt-Salze enthaltenden, Chitosan/Polyampholytlösungen werden durch Filtration gereinigt und danach bei 30 Prozent relativer Luftfeuchte auf speziell vorbereiteten Glasplatten 1 Woche bei Raumtemperatur (20 Grad Celsius) eingetrocknet. Danach wird die Filmqualität beurteilt.

Die Eigenschaften der relativ spröden bekannten Chitosansalz-Filme und des klebrigen Polymerfilms werden hier in vorteilhafter Weise kombiniert. Die das erfindungsgemäße Chitosan/Polyampholyt-Salz enthaltende Lösung C ergibt klare, glatte Filme, die in feuchtem Klima nicht klebrig (Vergleichsbeispiel B) werden, andererseits aber in trockenem Klima nicht brüchig und spröde (Vergleichsbeispiel A) sind.

**Tabelle 1**

| Kationischer Anteil des Polyampholyten (in Molprozent) | Grenzviskositätszahl $\eta$ [ml/g] 1) | Gehalt an Säuregruppen pro Gramm Polyampholytlösung (in mmol $H^+$) 2) | Trockensubstanzgehalt der wäßrigen Polyampholytlösung (in Gewichtsprozent) 3) | zur Bildung eines 1 : 1 Salzes erforderliche Menge Polyampholytlösung pro Gramm Chitosan (in Gramm) 4) | Löslichkeit von Chitosan in der Polyampholytlösung |
|---|---|---|---|---|---|
| 0 | 14,3 | 5,82 | 40 | 1,07 | unlöslich |
| 50 | 3,3 | 2,77 | 40 | 2,24 | bedingt löslich; starke milchige Trübung |
| 70 | 4,6 | 1,71 | 41 | 3,62 | mit schwacher Trübung löslich |
| 80 | 5,9 | 1,16 | 42 | 5,35 | klar löslich (geringer Rückstand) |
| 90 | 7,4 | 0,62 | 42 | 10,02 | klar löslich (geringer Rückstand) |

1) Die Ermittlung der Grenzviskositätszahl $\eta$ erfolgte mit einem DIN-Ubbelohde-Kapillarviskosimeter in einer wäßrigen Lösung von 0,2 mol/l Essigsäure und 0,1 mol/l Natriumchlorid bei 25 Grad Celsius.
2) Der Gehalt an Säuregruppen pro Gramm Polyampholytlösung wurde durch eine Säure-Base Titration mit 0,1n NaOH ermittelt.
3) Der Trockensubstanzgehalt wurde durch Ermittlung des Gewichtsverlustes nach 2 Stunden bei 105

Grad Celsius berechnet.

4) Als Chitosan wurde ein zu 90 Prozent deacetyliertes Chitin (mit 5,6 mmol freien Aminogruppen pro Gramm Chitosan) verwendet.

**Tabelle 2**

| Vergleichsbeispiel | A. | B. | C. |
|---|---|---|---|
| Lösung (in Wasser) von | 0,7 Prozent Chitosan und 1,8 Prozent Ameisensäure (10-prozentig) | 2 Prozent Polyampholyt (mit 70 Molprozent kationischem Anteil) | 0,48 Prozent Chitosan und 1,75 Prozent Polyampholyt (mit 70 Molprozent kationischem Anteil) |
| Filmbeurteilung | trüber, milchiger, spröder Film | klarer, glatter, relativ weicher Film (klebrig) | klarer, glatter Film |
| Pendelhärte nach König[1] | | | |
| a) bei 30 Prozent relativer Feuchte | 180 sec. | 61 sec. | 172 sec. |
| b) bei 70 Prozent relativer Feuchte | 142 sec. | 2 sec. | 17 sec. |

1) W. König, "Härtemessungen mit dem Pendelhärteprüfer", Farbe und Lack **65**, Seite 435 bis 443 (1959); DIN 53 157

**Beispiel 3** Haarfestiger, alkoholfrei

| | |
|---|---|
| 0,70 g | Chitosan |
| 2,53 g | 41-prozentige wäßrige Lösung eines Polyampholyten der Formel (I) mit einem kationischen Anteil von 70 Molprozent und einer Grenzviskositätszahl von 4,6 [ml/g] |
| 0,75 g | Parfümöl |
| 0,10 g | Formaldehyd (25-prozentige, wäßrige Lösung) |
| 95,92 g | Wasser |
| 100,00 g | |

20 ml dieser Lösung werden auf gewaschenes, handtuchtrockenes Haar verteilt, das Haar in üblicher Weise zur Frisur eingelegt und getrocknet. Bei guter Festigungswirkung zeigt das Haar im Vergleich zu einem Haarfestiger auf der Basis von Chitosan/Ameisensäure einen angenehmeren, elastischen und weicheren Griff.

**Beispiel 4** Tönungsfestiger

| | |
|---|---|
| 0,60 g | Chitosan |
| 15,03 g | 42-prozentige wäßrige Lösung eines Polyampholyten der Formel (I) mit einem kationischen Anteil von 90 Molprozent und einer Grenzviskositätszahl von 7,4 [ml/g] |
| 0,05 g | Acid Brown 4 (C.I. 41 805) |
| 84,32 g | Wasser |
| 100,00 g | |

20 ml dieser Lösung werden auf dem gewaschenen, handtuchtrockenen Haar verteilt und das Haar in üblicher Weise eingelegt und getrocknet. Das Haar besitzt anschließend eine leichte Rot-Braunfärbung und ist gefestigt.

**Beispiel 5** Fönlotion

| | | |
|---|---|---|
| 0,240 | g | Chitosan |
| 0,875 | g | Polyampholytlösung aus Beispiel 3 |
| 35,000 | g | Isopropanol |
| 0,500 | g | Parfüm |
| 0,100 | g | Farbe |
| 63,285 | g | Wasser |
| 100,000 | g | |

20 g dieser Lösung werden auf das gewaschene, handtuchtrockene Haar verteilt. Anschließend wird das Haar in üblicher Weise gefönt. Dabei zeigt das Haar ein hervorragendes Gleitvermögen der Bürste und ist bei sehr gutem Glanz hervorragend konditioniert.

**Beispiel 6** Einlegemittel mit extra starker Festigung

| | | |
|---|---|---|
| 1,50 | g | Chitosan |
| 15,03 | g | Polyampholytlösung aus Beispiel 4 |
| 0,80 | g | Parfümöl |
| 0,10 | g | Formaldehyd (25-prozentige wäßrige Lösung) |
| 82,57 | g | Wasser |
| 100,00 | g | |

20 ml dieser Lösung werden auf dem gewaschenen, handtuchtrockenen Haar verteilt. Anschließend wird das Haar in üblicher Weise zur Frisur eingelegt und getrocknet. Bei sehr starker Festigung zeigt das Haar im Vergleich zu Einlegemitteln auf der Basis von Salzen des Chitosans mit aliphatischen organischen Säuren einen angenehmeren, elastischeren und weicheren Griff.

**Beispiel 7** Haarkurmittel

| | |
|---|---|
| 0,30 g | Chitosan |
| 3,01 g | Polyampholytlösung aus Beispiel 4 |
| 3,00 g | Stearylalkohol |
| 2,00 g | Tris-(oligooxyethyl)-octadecyl-ammonium phosphat |
| 1,00 g | Wollwachs (Adeps Lanae) |
| 1,00 g | Vaseline |
| 89,69 g | Wasser |
| 100,00 g | |

35 g des Haarkurmittels werden im gewaschenen Haar verteilt und nach einer Einwirkzeit von 3 bis 5 Minuten mit Wasser wieder ausgespült. Als Ergebnis werden ausgezeichneter Griff, Glanz sowie Kämmbarkeit des Haares erhalten.

**Beispiel** 8 Haarwaschmittel

| | |
|---|---|
| 2,00 g | Chitosan |
| 9,52 g | Polyampholytlösung aus Beispiel 3 |
| 50,00 g | Dimethyl-carboxymethylen-propylenamidostea-rat-betain (30-prozentige wäßrige Lösung) |
| 38,48 g | Wasser |
| 100,00 g | |

Es wird ein klares Shampoo erhalten. Das damit gewaschene Haar ist hinsichtlich Glanz, Griff und Kämmbarkeit ausgezeichnet konditioniert.

**Beispiel** 9 Hautcreme

| | |
|---|---|
| 0,30 g | Chitosan |
| 3,00 g | Polyampholytlösung aus Beispiel 4 |
| 3,00 g | Stearylalkohol |
| 1,00 g | Wollwachs (Adeps Lanae) |
| 1,00 g | Vaseline |
| 1,00 g | Natriumacetylstearylsulfat |
| 90,70 g | Wasser, vollentsalzt |
| 100,00 g | |

Die kosmetischen Mittel gemäß den Beispielen 3 bis 9 werden wie folgt zubereitet:

Das Chitosan wird in der wäßrigen Polyampholytlösung gelöst. Anschließend wird die so erhaltene Chitosan/Polyampholytlösung mit den übrigen Bestandteilen des kosmetischen Mittels vermischt (Beispiel 3, 4, 5, 6 und 8) beziehungsweise emulgiert (Beispiel 7 und 9).

Die Messung der Grenzviskositätszahlen erfolgte in einer wäßrigen Lösung von 0,2 mol/l Essigsäure und 0,1 mol/l Natriumchlorid bei 25 Grad Celsius unter Verwendung eines DIN-Ubbelohde-Kapillarviskosimeters.

Die Prozentangaben sollen, sofern nicht anders angegeben, Gewichtsprozente darstellen.

**Patentansprüche**

1. Kosmetisches Mittel zur Behandlung der Haare und/oder der Haut auf der Basis von 0,05 bis 10 Gewichtsprozent Chitosan, dadurch gekennzeichnet, daß es 0,05 bis 20 Gewichtsprozent eines ampholytischen Copolymerisates der Formel (I)

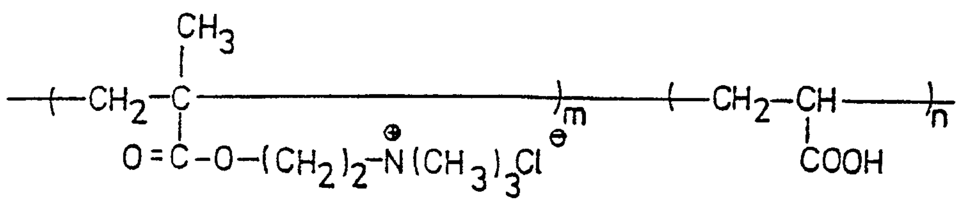
(I) ,

wobei m = 0,5 bis 0,9,
n = 0,1 bis 0,5 und
m + n = 1 ist,
enthält.

2. Mittel nach Anspruch 1, dadurch gekennzeichnet, daß es das Chitosan und das ampholytische Copolymerisat der Formel (I) in einer Gesamtmenge von 0,1 bis 10 Gewichtsprozent enthält.

3. Mittel nach Anspruch 1 und 2, dadurch gekennzeichnet, daß das Verhältnis von Chitosan (bezogen auf seinen Gehalt an freien Aminogruppen) und ampholytischem Copolymerisat (bezogen auf seinen anionischen Anteil) molar 1 : 1 bis 1 : 5 beträgt.

4. Mittel nach Anspruch 1 bis 3, dadurch gekennzeichnet, daß es in Form einer wäßrigen oder wäßrigalkoholischen Zubereitung, insbesondere als Lösung, Creme, Gel, Dispersion oder Emulsion, vorliegt.

5. Mittel nach Anspruch 1 bis 4, dadurch gekennzeichnet, daß der pH-Wert 2 bis 11 beträgt.

6. Mittel nach Anspruch 1 bis 5, dadurch gekennzeichnet, daß es zusätzlich mindestens einen kosmetischen Farbstoff in einer Konzentration von 0,01 bis 2,0 Gewichtsprozent enthält und in Form eines Farbfestigers oder Tönungsfestigers vorliegt.

7. Mittel nach Anspruch 1 bis 6, dadurch gekennzeichnet, daß es zusätzlich mindestens ein kationisches, anionisches, nichtionisches oder amphoteres Tensid enthält und in Form eines Haarwaschmittels vorliegt.

8. Mittel nach Anspruch 7, dadurch gekennzeichnet, daß es das Tensid in einer Menge von 3 bis 50 Gewichtsprozent enthält und einen pH-Wert zwischen 3 und 9 aufweist.

9. Mittel nach Anspruch 1 bis 8, dadurch gekennzeichnet, daß es als Kosmetikgrundlage eine wäßrige oder wäßrig-alkoholische Zubereitung enthält, die mit einem unter Druck verflüssigten Treibmittel vermischt ist, in einem Druckbehälter abgefüllt ist und in Form eines Aerosolsprays oder -schaums vorliegt.

10. Chitosan/Polyampholyt-Salz, erhältlich durch Umsetzung von Chitosan oder dessen wasserlöslichen Salzen mit einer äquimolaren Menge einer wäßrigen Lösung eines ampholytischen Copolymerisates der Formel (I).

**Claims**

1. Cosmetic composition for the treatment of the hair and/or the skin based on from 0.05 to 10 wt.% of chitosan, characterised in that it contains from 0.05 to 20 wt.% of an ampholytic copolymer of the formula (I)

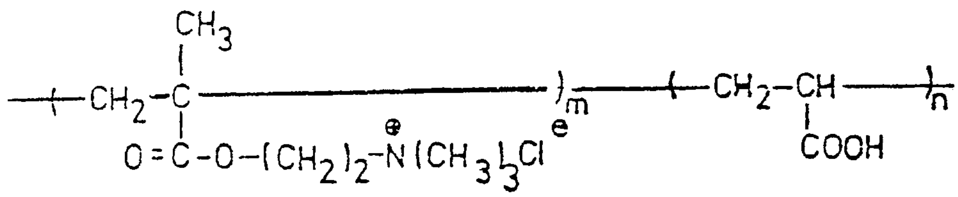

(I),

in which m = 0.5 to 0.9 and n = 0.1 to 0.5 and m + n = 1.

2. Composition according to Claim 1, characterised in that it contains the chitosan and the ampholytic copolymer of formula (I) in a total amount of from 0.1 to 10 wt.%.

3. Composition according to Claim 1 and 2, characterised in that the molar ratio of chitosan (based on its content of free amino groups) to ampholytic copolymer (based on its anionic part) is from 1:1 to 1:5.

4. Composition according to Claims 1 to 3, characterised in that it is in the form of an aqueous or aqueous/alcoholic preparation, especially in the form of a solution, cream, gel, dispersion or emulsion.

5. Composition according to Claims 1 to 4, characterised in that the pH value is from 2 to 11.

6. Composition according to Claims 1 to 5, characterised in that there is additionally present at least one cosmetic dye in a concentration of from 0.01 to 2.0 wt.% and it is in the form of a colour fixer or toning fixer.

7. Composition according to Claim 1 to 6, characterized in that it additionally contains a cationic, anionic, nonionic or amphoteric surface active agent in the form of a hair shampoo.

8. Composition according to Claim 7, characterised in that it contains the surface-active agent in an amount of from 3 to 50 wt.% and that it has a pH value between 3 and 9.

9. Composition according to Claims 1 to 8, characterised in that it contains an aqueous or aqueous-alcoholic preparation as a cosmetic base, which is mixed with a propellant that is liquefied under pressure, and charged into a pressure container and is in the form of an aerosol spray or aerosol foam.

10. Chitosan/polyampholytic salt, which can be obtained by the mixing of chitosan or a water-soluble salt thereof with an equimolar amount of an aqueous solution of an ampholytic copolymer of the formula (I).

**Revendications**

1. Composition cosmetique pour le traitement des cheveux et/ou de la peau, à base de 0,05 à 10 % en poids de chitosane, caractérisée en ce qu'elle comprend 0,05 à 20 % en poids d'un copolymerisat ampholytique de formule (I)

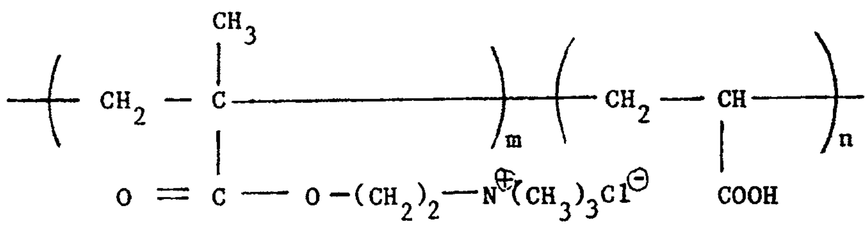

(I),

où m = 0,5 à 0,9
n = 0,1 à 0,5 et
m + n = 1.

2. Composition selon la revendication 1, caractérisée en ce qu'elle contient le chitosane et le copolymérisat de formule (I) dans une quantité totale de 0,1 à 10 % en poids.

3. Composition selon les revendications 1 et 2, caractérisée en ce que le rapport molaire du chitosane (rapporté à sa teneur en groupe Amino libres) et de copolymérisat ampholytique (rapporté à sa composante anionique) est de 1 : 1 à 1 : 5.

4. Composition selon les revendications 1 à 3, caractérisée en ce qu'elle existe sous forme d'une préparation aqueuse ou aqueuse-alcoolique, notamment d'une solution, d'une crème, d'un gel, d'une dispersion ou d'une émulsion.

5. Composition selon les revendications 1 à 4, caractérisée en ce que le pH va de 2 à 11.

6. Composition selon les revendications 1 à 5, caractérisée en ce qu'elle contient en outre au moins un colorant cosmétique d'une concentration de 0,01 % à 2,0 % en poids et existe sous forme d'un renforçateur de couleur ou de nuance.

7. Composition selon la revendication 1 à 6, caractérisée en ce qu'elle comporte en outre au moins un agent tensioactif cationique, anionique, non ionique ou amphotère et se présente sous forme d'un shampooing.

8. Composition selon la revendication 7, caractérisée en ce qu'elle contient l'agent tensioactif dans une quantité de 3 à 50 % en poids et présente un pH entre 3 et 9.

9. Composition selon les revendications 1 à 8, caractérisée en ce qu'elle contient une préparation aqueuse ou aqueuse-alcoolique comme base cosmétique mélangée à un agent moussant liquéfié sous pression, remplissant un récipient sous pression et se présentant sous la forme d'un aérosol pulvérisable ou en mousse.

**10.** Sel chitosane polyampholyte obtenu par transformation du chitosane ou de ses sels solubles dans l'eau avec une quantité équimolaire d'une solution aqueuse d'un copolymérisat ampholytique de formule (I).